Europäisches Patentamt
European Patent Office
Office européen des brevets

(19)

(11) Publication number: **0 498 889 A1**

(12) **EUROPEAN PATENT APPLICATION published in accordance with Art. 158(3) EPC**

(21) Application number: **90916059.0**

(51) Int. Cl.$^{5}$: **C12N 11/00**

(22) Date of filing: **31.10.90**

(86) International application number: **PCT/JP90/01395**

(87) International publication number: **WO 91/06640 (16.05.91 91/11)**

(30) Priority: **01.11.89 JP 287049/89**
**13.07.90 JP 185919/90**

(43) Date of publication of application: **19.08.92 Bulletin 92/34**

(84) Designated Contracting States: **AT BE CH DE DK ES FR GB GR IT LI LU NL SE**

(71) Applicant: **NIPPON SHINYAKU CO., LTD.**
**14, Kisshoinnishinoshomonguchicho**
**Minami-ku Kyoto-shi Kyoto 601(JP)**

(72) Inventor: **MARUO, Shigeaki**
**2-2 13-104, Minamiai Ibaraki-shi**
**Osaka 567(JP)**
Inventor: **MIYAZAKI, Katsunori**
**152, Takeyamachi Oshikojidori Takadura**
**Higashiiru**
**Nakagyo-ku Kyoto-shi Kyoto 604(JP)**
Inventor: **YAMADA, Naoyoshi**
**29-4-204, Oyake Sakanotsujicho**
**Yamashina-ku**
**Kyoto-shi Kyoto 607(JP)**
Inventor: **EZURE, Yohji**
**2-21-22, Nogohara Otsu-shi**
**Shiga 520-21(JP)**

(74) Representative: **Kügele, Bernhard et al**
**c/o NOVAPAT-CABINET CHEREAU 9, Rue du**
**Valais**
**CH-1202 Genève(CH)**

(54) **STABILIZED IMMOBILIZED ENZYME.**

(57) A technology for effectively stabilizing immobilized enzymes related to carbonhydrates, by immobilizing an enzyme stabilizer together with an enzyme on a carrier. The stabilizer includes a compound represented by general formula (1), (wherein R represents hydrogen, a lower alkyl, a hydroxyalkyl, a phenylalkyl, a phenylalkenyl, a phenylalkynyl, a phenoxyalkyl, a phenoxyalkenyl or a phenoxyalkynyl, each of the phenyl groups being optionally substituted, and X represents hydrogen or an axial or equatorial hydroxyl group), a glucose oligomer thereof and an α-cyclodextrin. According to the invention, the amount of the stabilizer can be minimized and the stabilizer can readily be recovered from the reaction mixture.

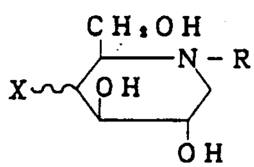
〔I〕

EP 0 498 889 A1

Detailed Description of the Invention

[Industrial Field of Utilization]

This invention relates to a technology for stabilizing, to an increased extent, immobilized enzymes acting on carbohydrates, such as cyclodextrin glycosyl transferase (CGTase), glucoamylase (GA), β-amylase, β-glucosidase (GD) and β-galactosidase.

Nowadays enzymes acting on carbohydrates are widely used in the chemical industry. For example, CGTase is an enzyme generally used in commercial scale cyclodextrin production from starch. Cyclodextrins are substances essential for the production of clathrate compounds for improving the stability of drugs or masking odor-emitting substances. Therefore a technology for stabilizing CGTase would have a very high industrial significance.

GA and β-amylase are in daily use in producing glucose or maltose from starch, hence a technology for stabilizing GA and β-anylase would have a very high significance.

Furthermore, β-glucosidase is an enzyme playing an important role in wood saccharification and stable grades thereof have been sought after.

[Prior Art]

Marked advances have been made recently in the enzymatic reaction technology, giving a "poles apart" feeling. In particular, the immobilized enzyme technology has made it possible, with favorable results, to conduct enzymatic reactions efficiently and with reduced amounts of enzymes as compared with the earlier conventional processes comprising conducting enzymatic reactions in the solution phase.

A plurality of patent applications have been published concerning the use of chitosan beads in immobilized enzyme processes. Japanese Kokai Tokkyo Koho No. 63-196290, for instance, discloses a technique comprising immobilizing cyclodextrin glucanotransferase on porous chitosan beads and then further treating the beads with a crosslinking agent.

Efficient application of the above-mentioned enzymes had been the subject of a number of investigations. As regards the stabilization of enzymes themselves, however, the study results have not always been satisfactory. Enzymes lose their activity gradually as they are used. Under ordinary conditions, CGTase, for instance, loses one half its initial activity in several days and becomes quite inactive after the lapse of a few scores of days.

Even in the case of immobilized enzymes, once their activity has been lost, there is no other way but to supply fresh portions of the enzymes. Meanwhile the present inventors previously found that this technological problem can be fairly solved when enzymatic reactions are carried out in the presence of a certain substance capable of stabilizing enzymes in a relatively small amount (hereinafter referred to as "stabilizing agent") (Japanese Patent Application No. 033481/1989).

[Problems Which the Invention is to Solve]

The technique just mentioned above can indeed realize enzyme stabilization but requires the use of such stabilizing agent in a fairly large feeding amount since the stabilizing agent must be fed in a state such that it can be in contact with the enzyme on the occasion of enzymatic reaction. Furthermore it is necessary to recover the stabilizing agent from the reaction mixture after completion of the reaction.

It is an object of the present invention to solve the technological problems mentioned above.

[Means for Solving the Problems]

The invention provides a stabilized immobilized enzyme which comprises a carrier, a enzyme acting on carbohydrates and immobilized on said carrier and an enzyme stabilizing agent immobilized on said carrier together with said enzyme.

The immobilized enzyme having the above-mentioned constitution has been created for the first time by the present inventors.

In the following, the constitution of the present invention is described in detail.

The carrier to be used in the practice of the invention may be any carrier suited for immobilizing an enzyme acting on carbohydrates thereon and includes, among others, chitosan beads, which are generally used nowadays in producing immobilized enzymes, agarose resins and rigid synthetic resins which are porous. Said resins may be in the form of beads or flat membranes.

As typical grades of chitosan beads which can be used in the practice of the invention, there may be mentioned Chitopearl BCW-1000 series chitosan beads (commercially available from Fuji Spinning Co.).

As an agarose resin usable in the practice of the invention, there may be mentioned Sepharose 6B (Pharmacia), among others.

As examples of the rigid, porous synthetic resins which are usable in the practice of the invention, there may be mentioned those hydrophilic porous polymers that are commercially available from Japan Organo Co. under the designations EF-4611 and EF-4612.

In the practice of the invention, the carrier is first reacted with a crosslinking agent for forming active linking sites on the carrier surface and then the enzyme specified herein is immobilized on said carrier at said sites, namely at crosslinking agent ends.

In accordance with one aspect of the invention, a stabiling agent can be immobilized on the carrier at crosslinking agent ends simultaneously with the enzyme immobilization.

In accordance with another aspect of the invention, it is also possible to first immobilize the enzyme on the carrier at crosslinking agent ends and then immobilize the stabilizing agent on the same carrier at remaining crosslinking agent ends, and vice versa.

Usable as the crosslinking agent are oxirane and other epoxide-containing carbon chains as well as those crosslinking agents that are generally used in preparing immobilized enzymes, for example glutaraldehyde and the like.

When CGTase is taken as an example of the enzyme, the stabilized immobilized enzyme according to the invention can be produced, for example, by adding an appropriate carrier (e.g. chitosan beads) to an appropriate alkaline solution (e.g. 0.6 N sodium hydroxide) at an appropriate temperature (e.g. room temperature), then adding an appropriate crosslinking agent (e.g. oxirane) to an appropriate concentration (e.g. equivalent to the alkali), shaking the resulting mixture gently at an appropriate temperature (e.g. 8° to 50°C) for an appropriate period (e.g. 1 to 4 hours), filtering the mixture, washing the carrier with water, adding to the carrier in water or an appropriate buffer (e.g. 0.025 M acetate buffer, pH 6.0), a CGTase solution with an appropriate concentration (e.g. 1 to 500 mg/ml) and simultaneously an appropriate stabilizing agent (e.g. glucosylmoranoline) to an appropriate concentration (e.g. 0.9 to 1 g/ml), shaking the resulting mixture gently for an appropriate period (e.g. 1 to 48 hours), then filtering the mixture and washing the solid phase with water.

The above-mentioned process has been established for the first time by the present inventors.

As examples of the stabilizing agent which are particularly suited for use in the practice of the invention, there may be mentioned compounds of the general formula

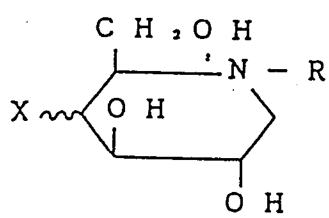

(I)

wherein R is hydrogen, lower alkyl, hydroxyalkyl, phenylalkyl, phenylalkenyl, phenylalkynyl, phenoxyalkyl, phenoxyalkenyl or phenoxyalkynyl, where each phenyl moiety may be substituted phenyl or unsubstituted phenyl, and X is hydrogen or axial or equatorial hydroxyl, and glucose oligomer derivatives thereof.

The compounds of general formula [I] are known in the art and can be prepared, for example, by the methods disclosed in Japanese Patent Publications Nos. 56-099195, 60-2038, 61-2076 and 62-242691.

The glucose oligomer derivatives mentioned above may be represented by the general formula

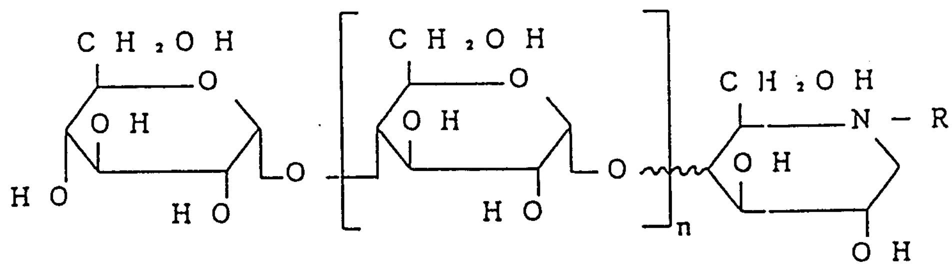

[II]

wherein R is as defined above and n is an integer of 0 to 24.

The lower alkyl represented by R in general formula [I] and in general formula [II] is, for example, methyl, ethyl, n-propyl, isopropyl, n-butyl, isobutyl, sec-butyl or tert-butyl.

The alkyl moiety of the hydroxyalkyl may contain 1 to 5 carbon atoms.

The alkyl moiety of the phenylalkyl may contain 1 to 5 carbon atoms and the phenyl moiety thereof may be substituted or unsubstituted phenyl.

The alkenyl moiety of the phenylalkenyl may contain 2 to 5 carbon atoms and the phenyl moiety thereof may be substituted phenyl or unsubstituted phenyl.

The alkynyl moiety of the phenylalkynyl may contain 2 to 5 carbon atoms and the phenyl moiety thereof may be substituted phenyl or unsubstituted phenyl.

The alkyl moiety of the phenoxyalkyl may contain 1 to 5 carbon atoms and the phenyl moiety thereof may be substituted phenyl or unsubstituted phenyl.

The alkenyl moiety of the phenoxyalkenyl may contain 2 to 5 carbon atoms and the phenyl moiety thereof may be substituted phenyl or unsubstituted phenyl.

The alkynyl moiety of the phenoxyalkynyl may contain 2 to 5 carbon atoms and the phenyl moiety thereof may be substituted phenyl or unsubstituted phenyl.

The stabilizing agent to be used in accordance with the invention also includes; in addition to those mentioned above α-cyclodextrin, galactostatin and derivatives thereof and the like.

For enzyme stabilization in accordance with the invention, it is recommendable that immobilization is conducted in a state such that 1 to 50 mg, for instance, of enzyme is adsorbed on each gram of carrier and 10 μg to 6 mg, for instance, of stabilizing agent is present per gram of carrier. The amount of stabilizing agent may suitably be varied depending on the intended purpose.

As is evident from the examples given below, the amount of stabilizing agent required in practicing the invention is much smaller than in the conventional methods. This is one of the characteristic features of the present invention.

[Examples]

The following examples are further illustrative of the stabilization method according to the invention.

Reference Example 1

Epoxy-activated Sepharose beads

Sepharose 6B was placed on a glass filter and suctioned for filtration and the matter on the filter was suction-dried. To 10 g of the thus-dried Sepharose were added 10 ml of oxirane (Aldrich) and 10 ml of 0.6 N sodium hydroxide. The mixture was shaken gently at room temperature for 16 hours for effecting the reaction. The beads were recovered and thoroughly washed with water. The thus-obtained beads were used as epoxy-activated Sepharose beads.

Reference Example 2

Epoxy-activated chitosan beads

Chitosan beads (ø 1.5,mm, 10 ml) were placed on a glass filter and thoroughly washed with water. Oxirane (10 ml) and 10 ml of 0.6 N sodium hydroxide were added to the beads and the mixture was shaken gently at room temperature for 16 hours. After the reaction effected in this way, the beads were thoroughly washed with water and used as epoxy-activated chitosan beads.

Example 1

To 3 g of epoxy-activated Sepharose beads was added 15 ml of 0.1 N sodium hydroxide and further 60 mg of moranoline. The mixture was shaken gently at 40 °C for 1 hour, the beads were then thoroughly washed with water on a glass filter and dispersed in 15 ml of water, 60 mg of powdery β-glucosidase (almond-derived, Sigma) was added, and the mixture was shaken gently at 40 °C for 20 hours for effecting the reaction. The moranoline and β-glucosidase-bound Sepharose beads finally obtained were thoroughly washed with water.

Example 2

Epoxy-activated Sepharose beads (2 g) were dispersed in 10 ml of water. N-Methylmoranoline (2 g) and 40 mg of β-glucosidase (Sigma) were added to the dispersion, and the mixture was shaken gently at 40 °C for 20 hours. The beads were recovered by filtration using a glass filter and thoroughly washed with water.

Example 3

Epoxy-activated Sepharose beads (2 g) were dispersed in 10 ml of water. Moranoline (500 mg) and 30 mg of glucoamylase (derived from Rhizopus niveus; Seikagaku Kogyo) were added to the dispersion, the mixture was shaken gently at room temperature for 20 hours and then the beads were recovered on a glass filter and thoroughly washed with water.

Example 4

Epoxy-activated chitosan beads (5 ml) were dispersed in 10 ml of water. Glucoamylase (derived from Aspergillus; Glucozyme NL-3, Amano Pharmaceutical) (500 µl) and 500 mg of moranoline were added to the dispersion. The mixture was shaken gently at room temperature for 20 hours. After the reaction, the beads were collected on a glass filter and thoroughly washed with water.

Example 5

Epoxy-activated chitosan beads (5 ml) were dispersed in 5 ml of 0.1 N sodium hydroxide, 1 g of N-(2-hydroxyethyl)-moranoline was added to the dispersion, and the mixture was shaken gently at 40 °C for 20 hours for effecting the reaction. The beads were recovered on a glass filter and thoroughly washed with water. These beads were dispersed in 5 ml of water, 1 ml of glucoamylase (Amano Pharmaceutical) was added to the dispersion, and the mixture was shaken gently at room temperature for 16 hours for effecting the reaction. The beads were collected on a glass filter and thoroughly washed with water.

Example 6

To 5 g of epoxy-activated Sepharose beads was added 50 ml of 0.1 N sodium hydroxide for dispersing the beads, 3 g of α-cyclodextrin was dissolved in the dispersion, and the resultant dispersion was shaken gently at 40 °C for 24 hours. The beads were recovered on a glass filter, thoroughly washed with water and then dispersed in 10 ml of water. Glucoamylase (30 mg; Seikagaku Kogyo) was dissolved in the dispersion, the mixture was shaken gently again at 40 °C for 20 hours for effecting the reaction, then collected on a glass filter and thoroughly washed with water.

Example 7

Epoxy-activated chitosan beads (10 ml) was added to 10 ml of 1 N sodium hydroxide, 10 g of glucosylmoranoline was dissolved in the dispersion, and the mixture was shaken gently at 40 °C for 16

hours. The beads were thoroughly washed on a glass filter and dispersed in 10 ml of water adjusted to pH 10 with aqueous sodium hydroxide, 10 ml of CGTase (derived from Bacillus stearothermophilus; Hayashibara Biochemical Labs.) was added, and the mixture was shaken gently at 40°C for 6 hours. After the reaction, the beads were recovered on a glass filter and thoroughly washed with water.

Example 8

Epoxy-activated chitosan beads (10 ml) was added to 10 ml of water, 2.5 g of glucosyl-N-methylmoranoline was dissolved in the dispersion, 0.3 ml of CGTase was further added, and the resultant mixture was shaken gently at 40°C for 16 hours for effecting the reaction. The beads were then recovered on a glass filter and thoroughly washed with water.

Example 9

Epoxy-activated chitosan beads (3 ml) was added to 5 ml of water, 250 mg of 1-deoxygalactostatin and 20 mg of β-galactosidase (derived from Rhizopus; Toyobo) were dissolved in the dispersion. The mixture was shaken gently at 25°C for 16 hours for effecting the reaction. The beads were then recovered on a glass filter and thoroughly washed with water.

Example 10

Epoxy-activated chitosan beads (3 ml) was added to 5 ml of water, 500 mg of 1,4-dideoxygalactostatin and 20 mg of β-galactosidase (Toyobo) were dissolved in the dispersion. The resulting mixture was shaken gently at 25°C for 16 hours for effecting the reaction. The beads were then collected on a glass filter and thoroughly washed with water.

Example 11

Epoxy-activated chitosan beads (3 ml) was added to 5 ml of 0.1 N sodium hydroxide for dispersion. Glucosylmoranoline (500 mg) was dissolved in the dispersion and the resultant mixture was shaken gently at 37°C for 16 hours for effecting the reaction. The beads were recovered on a glass filter and thoroughly washed with water. A β-amylase extract [5 ml; prepared by extracting 10 g of β-amylase #1500 (Amano Pharmaceutical) with 70 ml of water] was added to the beads and the mixture was shaken gently again at room temperature for 16 hours for effecting the reaction. Then the beads were recovered on a glass filter and thoroughly washed with water.

[Test Examples]

The method used for evaluation of the stabilizing effect of the invention is as follows. The beads of the invention and the control beads were respectively taken in such amounts that as determined at the temperature of 37-40°C which is generally recommended for assay of this enzyme, the two kinds of beads would show substantially the same degree of activity and after transfer of the beads into a high-temperature environment, the patterns of loss of activity from the respective beads were compared.

Test Example 1

The substance according to the invention as obtained in Example 1 (50 mg) and 200 mg of the substance (control) prepared in the same manner but without adding moranoline were respectively placed in test tubes and each dispersed in 10 ml of 0.12 M salicin solution (in 0.1 M acetate buffer, pH 5.0). Incubation was conducted at 58°C and sampling was repeated at timed intervals for assaying glucose liberated by the glucose oxidase method. The results obtained are shown in Fig. 1. It is evident that the substance according to the invention is superior in heat stability to the control.

Test Example 2

The substance according to the invention as obtained in Example 1 (25 mg) and 40 mg of the substance (control) prepared in the same manner but without adding moranoline were respectively placed in test tubes, 750 µl of 0.1 M acetate buffer (pH 5.0) was added to each tube. The tubes were heated at one

of several specified temperatures within the range of 40° to 75°C for 30 minutes. After heating, the tubes were cooled to room temperature, 250 µl of 0.12 M salicin solution (in 0.1 M acetate buffer, pH 5.0) was added to each tube. After 30 minutes of reaction at 30°C, the glucose liberated was assayed by the glucose oxidase method and the percentage of the color intensity to that obtained after heat treatment at 40°C was reported as "percent residual activity". The data thus obtained are graphically shown in Fig. 2. It is evident that the substance according to the invention is stable to heat.

Test Example 3

The substance according to the invention as obtained in Example 2 (20 mg) and 120 mg of the substance (control) prepared in the same manner but without adding N-methylmoranoline were respectively placed in test tubes and each dispersed in 10 ml of 0.12 M salicin solution (in 0.1 M acetate buffer, pH 5.0). Incubation was conducted at 58°C and sampling was repeated at timed intervals for assaying the liberated glucose by the glucose oxidase method. The results thus obtained are graphically shown in Fig. 3. It is evident that the substance according to the invention is superior in heat stability to the control.

Test Example 4

The substance according to the invention as obtained in Example 2 (6 mg) and 60 mg of the substance (control) prepared in the same manner but without adding N-methylmoranoline were respectively placed in test tubes, 750 µl of 0.1 M acetate buffer (pH 5.0) was added to each tube, and each mixture was heated at one of several specified temperatures within the range of 40° to 75°C for 30 minutes and then cooled to room temperature. To each tube was added 250 µl of 0.12 M salicin solution (in 0.1 M acetate buffer, pH 5.0), the reaction was allowed to proceed at 30°C for 30 minutes, and the liberated glucose was assayed by the glucose oxidase method. The percentage of the resulting color intensity to that obtained after heat treatment at 40°C was reported as "percent residual activity". The data thus obtained are graphically shown in Fig. 4. It is evident that the substance according to the invention is thermally stable.

Test Example 5

The substance according to the invention as obtained in Example 3 (60 mg) and 1 mg of the substance (control) prepared in the same manner but without adding moranoline were respectively placed in test tubes, 200 µl of 0.021 M p-nitrophenyl α-D-glucopyranoside and 800 µl of 0.1 M acetate buffer (pH 6.0) were added to each tube and incubation was performed at 55°C. After the lapse of a specified time, 2 ml of 0.1 M sodium carbonate was added to each tube, the mixture was centrifuged at 2,000 rpm for 1 minute, and the supernatant was measured for optical density at 400 nm. The results thus obtained are graphically shown in Fig. 5. It is evident that the substance according to the invention is thermally stable.

Test Example 6

The substance according to the invention as obtained in Example 4 (100 grains) and the substance (control) prepared in the same manner but without adding moranoline (one grain) were respectively placed in test tubes, 200 µl of 0.021 M p-nitrophenyl α-D-glucopyranosideand 800 µl of 0.1 M acetate buffer (pH 6.0) were added to each tube, and incubation was conducted at 60°C. After the lapse of a specified time, 2 ml of 0.1 M sodium carbonate was added to each tube and the optical density was measured at 400 nm. The data thus obtained are graphically shown in Fig. 6. It is evident that the substance according to the invention is thermally stable.

Test Example 7

The substance according to the invention as obtained in Example 5 (3 grains) and the substance (control) prepared in the same manner but without adding N-(2-hydroxyethyl)moranoline (2 grains) were respectively added to test tubes, 200 µl of 0.021 M p-nitrophenyl α-D-glucopyranoside and 800 µl of 0.1 M acetate buffer (pH 5.5) were added to each tube and incubation was conducted at 63°C. After the lapse of a specified time, 2 ml of 0.1 M sodium carbonate was added to each tube and the supernatant was adequately diluted and measured for optical density at 400 nm. The data obtained in this manner are graphically shown in Fig. 7. It is evident that the substance according to the invention is thermally stable.

Test Example 8

The substance according to the invention as obtained in Example 6 (1.5 mg) and 3 mg of the substance (control) prepared in the same manner but without adding α-cyclodextrin were respectively placed in test tubes, 200 µl of 0.021 M p-nitrophenyl α-D-glucopyranoside and 800 µl of 0.1 M acetate buffer (pH 6.0) were added to each tube and incubation was conducted at 55 °C. After the lapse of a specified time, 2 ml of 0.1 M sodium carbonate was added to each tube and the resultant mixture was centrifuged at 2,000 rpm for 1 minute. The supernatant was measured for optical density at 400 nm. The results thus obtained are graphically shown in Fig. 8. It is evident that the substance according to the invention is thermally stable.

Test Example 9

The substance according to the invention as obtained in Example 6 (10 mg) and 20 mg of the substance (control) prepared in the same manner but without adding α-cyclodextrin were respectively placed in test tubes, 1 ml of 0.05 M acetate buffer (pH 4.6) was added to each tube, and each mixture was heated at one of several specified temperatures within the range of 40° to 70 °C for 30 minutes and then cooled to room temperature. To each tube was added 1 ml of 10% maltose solution. The reaction was allowed to proceed at 40 °C for 20 minutes, then 2 ml of 0.1 N sodium hydroxide was added, a 100-µl portion of the resultant solution was taken and assayed for liberated glucose by the glucose oxidase method, and the percentage of the color intensity to that obtained after heat treatment at 40 °C was reported as "percent residual activity". The data thus obtained are shown in Fig. 9. It is evident that the substance according to the invention is thermally stable.

Test Example 10

The substance according to the invention as obtained in Example 7 (2 grains) and the substance (control) obtained in the same manner but without adding glucosylmoranoline (10 grains) were respectively placed in test tubes, 500 µl of 0.05 M phosphate buffer (pH 6.2) was added to each tube. Each tube was heated at one of several specified temperatures (60° to 75 °C) for 30 minutes and then cooled to room temperature. To the tube were added 250 µl of a solution α-cyclodextrin (20 mM) and sucrose (100 mM) in 0.2 M acetate buffer (pH 4.5) and 250 µl of a glucoamylase solution [prepared by dissolving 10 mg of glucoamylase (Seikagaku Kogyo) in 1.5 ml of 0.2 M acetate buffer (pH 4.5)]. The reaction was allowed to proceed at 40 °C for 30 minutes and the glucose liberated was assayed by the glucose oxidase method. The percentage of the color intensity to that obtained after heat treatment at 40 °C was reported as "percent residual activity". The data thus obtained are graphically shown in Fig. 10. It is evident that the substance according to the invention is thermally stable.

Test Example 11

The substance according to the invention as obtained in Example 8 (500 grains) and the substance (control) prepared in the same manner but without adding glucosyl-N-methylmoranoline (50 grains) were respectively placed in test tubes, 500 µl of 0.05 M phosphate buffer (pH 6.2) was added to each tube, and each tube was heated at one of several specified temperatures (60° to 75 °C) for 30 minutes and then cooled to room temperature. To each tube was added 250 µl of a solution of α-cyclodextrin (20 mM) and sucrose (100 mM) in 0.2 M acetate buffer (pH 4.5) and 250 µl of a glucoamylase solution [prepared by dissolving 10 mg of glucoamylase (Seikagaku Kogyo) in 1.5 ml of 0.2 M acetate buffer (pH 4.5)]. The reaction was allowed to proceed at 40 °C for 30 minutes and the glucose liberated was assayed by the glucose oxidase method. The percentage of the color intensity to that obtained after heat treatment at 40 °C was reported as "percent residual activity". The data thus obtained are shown in Fig. 11. It is evident that the substance according to the invention is thermally stable.

Test Example 12

The substance according to the invention as obtained in Example 9 (one grain) and the substance (control) prepared in the same manner but without adding 1-deoxygalactostatin (one grain) were respectively placed in test tubes and 1.5 ml of 0.02 M o-nitrophenyl β-D-galactopyranoside (in 0.1 M acetate buffer, pH 5.0) was added to each tube. Each mixture was incubated at 65 °C and, after the lapse of a specified time, 2 ml of 0.2 M sodium carbonate was added to each tube. The control mixture was further

diluted 10-fold with water depending on the intensity of color development. Optical density measurement was performed at 420 nm. The results thus obtained are shown in Fig. 12. It is evident that the substance according to the invention is thermally stable.

Test Example 13

The substance according to the invention as obtained in Example 9 (one grain) and the substance (control) prepared in the same manner but without adding 1-deoxygalactostatin (one grain) were respectively placed in test tubes, 1.5 ml of 0.1 M acetate buffer (pH 5.0) was added to each tube, and each tube was heated at one of several specified temperatures (50° to 75°C) for 30 minutes and then cooled to room temperature. To each tube was added 500 µl of 0.02 M o-nitrophenyl β-D-galactopyranoside solution (in 0.1 M acetate buffer, pH 5.0), and the reaction was allowed to proceed at 37°C for 15 minutes. 0.2 M sodium carbonate (2 ml) was added to each tube and the optical density was measured at 420 nm. The percentage of the color intensity to that obtained after heat treatment at 50°C was reported as "percent residual activity". The results thus obtained are shown in Fig. 13. It is evident that the substance according to the invention is thermally stable.

Test Example 14

The substance according to the invention as obtained in Example 10 (8 grains) and the substance (control) prepared in the same manner but without adding 1,4-dideoxygalactostatin (one grain) were respectively placed in test tubes and 500 µl of 0.02 M o-nitrophenyl β-D-galactopyranoside solution (in 0.1 M acetate buffer, pH 5.0) and 1.5 ml of 0.1 M acetate buffer (pH 5.0) were added to each tube. Each tube was incubated at 65°C and, after the lapse of a specified time, 2 ml of 0.2 M sodium carbonate was added to the tube and subjected to optical density measurement at 420 nm. The control was diluted 10-fold with water depending on the intensity of color development prior to optical density measurement. The results thus obtained are shown in Fig. 14. It is evident that the substance according to the invention is thermally stable.

Test Example 15

The substance according to the invention as obtained in Example 10 (8 grains) and the substance (control) prepared in the same manner but without adding 1,4-dideoxygalactostatin (one grain) were respectively placed in test tubes, 1.5 ml of 0.1 M acetate buffer (pH 5.0) was added to each tube, and each tube was heated at one of several specified temperatures (50° to 75°C) for 30 minutes and then cooled to room temperature. To each tube was added 500 µl of 0.02 M o-nitrophenyl β-D-galactopyranoside solution (in 0.1 M acetate buffer, pH 5.0). After the reaction was allowed to proceed at 37°C for 15 minutes, 2 ml of 0.2 M sodium carbonate was added to the tube and the optical density was measured at 420 nm. The percentage of the color intensity to that obtained after heat treatment at 50°C was reported as "percent residual activity". The results thus obtained are shown in Fig. 15. It is evident that the substance according to the invention is thermally stable.

Test Example 16

The substance according to the invention as obtained in Example 11 (2 grains) and the substance (control) prepared in the same manner but without adding glucosylmoranoline (10 grains) were respectively placed in test tubes and 2.5 ml of 0.02 M acetate buffer (pH 4.8) and 2.5 ml of 10% soluble starch solution were added to each tube. Incubation was conducted at 65°C and sampling was done in 50-µl portions at timed intervals. 3,5-Dinitrosalicylic acid solution (500 µl) was added to each sample, the mixture was heated in a boiling water bath for 5 minutes and then cooled with water, followed by addition of 5 ml of water. After thorough stirring, the resultant mixture was measured for optical density at 535 nm. The results thus obtained are shown in Fig. 16. It is evident that the substance according to the invention is thermally stable.

Test Example 17

The substance according to the invention as obtained in Example 11 (5 grains) and the substance (control) prepared in the same manner but without adding glucosylmoranoline (10 grains) were respectively placed in test tubes, 250 µl of 0.02 M acetate buffer (pH 4.8) was added to each tube, and each tube was

heated at one of several specified temperatures (50° to 75°C) for 30 minutes and then cooled to room temperature. To each tube was added 250 µl of 1% soluble starch solution, and the tube was further heated at 30°C for 30 minutes for effecting the reaction. Then, 1 ml of 3,5-dinitrosalicylic acid solution was added and the mixture was heated on a boiling water bath for 5 minutes. After cooling with water, 4.5 ml of water was added and the resultant mixture was stirred well and measured for optical density at 535 nm. The percentage of the color intensity to that at 50°C was calculated as "percent residual activity". The data thus obtained are diagrammatically shown in Fig. 17. It is evident that the substance according to the invention is thermally stable.

4. Brief Description of the Drawings

Fig. 1 shows the stability test results obtained in Test Example 1. [●, data for the substance according to the invention as obtained in Example 1; o, data for the substance for comparison prepared in the same manner but without adding moranoline; ordinate, optical density of solution; abscissa, time (hours).]
Fig. 2 shows the results obtained in Test Example 2. [●, data for the substance according to the invention as obtained in Example 1; o, data for the substance prepared in the same manner but without adding moranoline; oridnate, percent residual activity; abscissa, temperature (°C).]
Fig. 3 shows the results obtained in Test Example 3. [●, data for the substance according to the invention as obtained in Example 2; o, data for the substance for comparison prepared in the same manner but without adding N-methylmoranoline; ordinate, optical density of solution; abscissa, time (hours).]
Fig. 4 shows the results obtained in Test Example 4. [●, data for the substance according to the invention as obtained in Example 2; o, data for the substance prepared in the same manner but without adding N-methylmoranoline; ordinate, percent residual activity; abscissa, temperature (°C).]
Fig. 5 shows the results obtained in Test Example 5. [●, data for the substance according to the invention as obtained in Example 3; o, data for the substance prepared in the same manner but without adding moranoline; ordinate, optical density of solution; abscissa, time (hours).]
Fig. 6 shows the results obtained in Test Example 6. [●, data for the substance according to the invention as obtained in Example 4; o, data for the substance prepared in the same manner but without adding moranoline; ordinate, optical density of solution; abscissa, time (hours).]
Fig. 7 shows the results obtained in Test Example 7. [●, data for the substance according to the invention as obtained in Example 5; o, data for the substance prepared in the same manner but without adding N-(2-hydroxyethyl)moranoline; ordinate, optical density of solution; abscissa, time (hours).]
Fig. 8 shows the results obtained in Test Example 8. [●, data for the substance according to the invention as obtained in Example 6; o, data for the substance prepared in the same manner but without adding α-cyclodextrin; ordinate, optical density of solution; abscissa, time (hours).]
Fig. 9 shows the results obtained in Test Example 9. [●, data for the substance according to the invention as obtained in Example 6; o, data for the substance prepared in the same manner but without adding α--cyclodextrin; ordinate, percent residual activity; abscissa, temperature (°C).]
Fig. 10 shows the results obtained in Test Example
10. [●, data for the substance according to the invention as obtained in Example 7; o, data for the substance prepared in the same manner but without adding glucosylmoranoline; ordinate, percent residual activity; abscissa, temperature (°C).]
Fig. 11 shows the results obtained in Test Example 11. [●, data for the substance according to the invention as obtained in Example 8; o, data for the substance prepared in the same manner but without adding glucosylmoranoline; ordinate, percent residual activity; abscissa, temperature (°C).]
Fig. 12 shows the results obtained in Test Example 12. [●, data for the substance according to the invention as obtained in Example 9; o, data for the substance prepared in the same manner but without adding 1-deoxygalactostatin; ordinate, optical density of solution; abscissa, time (hours).]
Fig. 13 shows the results obtained in Test Example 13. [●, data for the substance according to the invention as obtained in Example 9; o, data for the substance prepared in the same manner but without adding 1-deoxygalactostatin; ordinate, percent residual activity; abscissa, temperature (°C).]
Fig. 14 shows the results obtained in Test Example 14. [●, data for the substance according to the invention as obtained in Example 10; o, data for the substance prepared in the same manner but without adding 1,4-dideoxygalactostatin; ordinate, optical density of solution; abscissa, time (hours).]
Fig. 15 shows the results obtained in Test Example 15. [●, data for the substance according to the invention as obtained in Example 10; o, data for the substance prepared in the same manner but without adding 1,4-dideoxyglactostatin; ordinate, percent residual activity; abscissa, temperature (°C).]

Fig. 16 shows the results obtained in Test Example 16. [●, data for the substance according to the invention as obtained in Example 11; o, data for the substance prepared in the same manner but without adding glucosylmoranoline; ordinate, optical density of solution; abscissa, time (hours).]

Fig. 17 shows the results obtained in Test Example 17. [●, data for the substance according to the invention as obtained in Example 11; o, data for the substance prepared in the same manner but without adding glucosylmoranoline; ordinate, percent residual activity; abscissa, temperature (° C).

## Claims

1. In immobilized enzyme wherein enzyme is immobilized on the surface of a carrier and the enzyme acts on carbohydrates, a stabilized immobilized enzyme, characterized in that, a substance capable of stabilizing said enzyme is simultaneously immobilized on said carrier.

2. A stabilized immobilized enzyme of claim 1 wherein the stabilizing substance is a compound expressed by the following general formula (I) or glucose oligomer thereof.

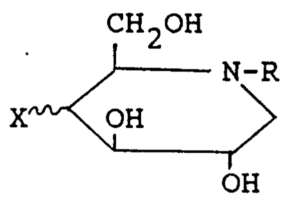

(I)

in which R is hydrogen, lower alkyl, hydroxyalkyl, phenylalkyl, phenylalkenyl, phenylalkynyl, phenoxyalkyl, phenoxyalkenyl or phenoxyalkynyl where "phenyl" stands for both substituted and unsubstituted ones and X is hydrogen or axial or equatorial hydroxyl.

3. A stabilized immobilized enzyme of claim 1 wherein the stabilizing substance is alpha-cyclodextrin.

Fig. 1 Heat stability of moranoline- and β-glucosidase-bound Sepharose 6B

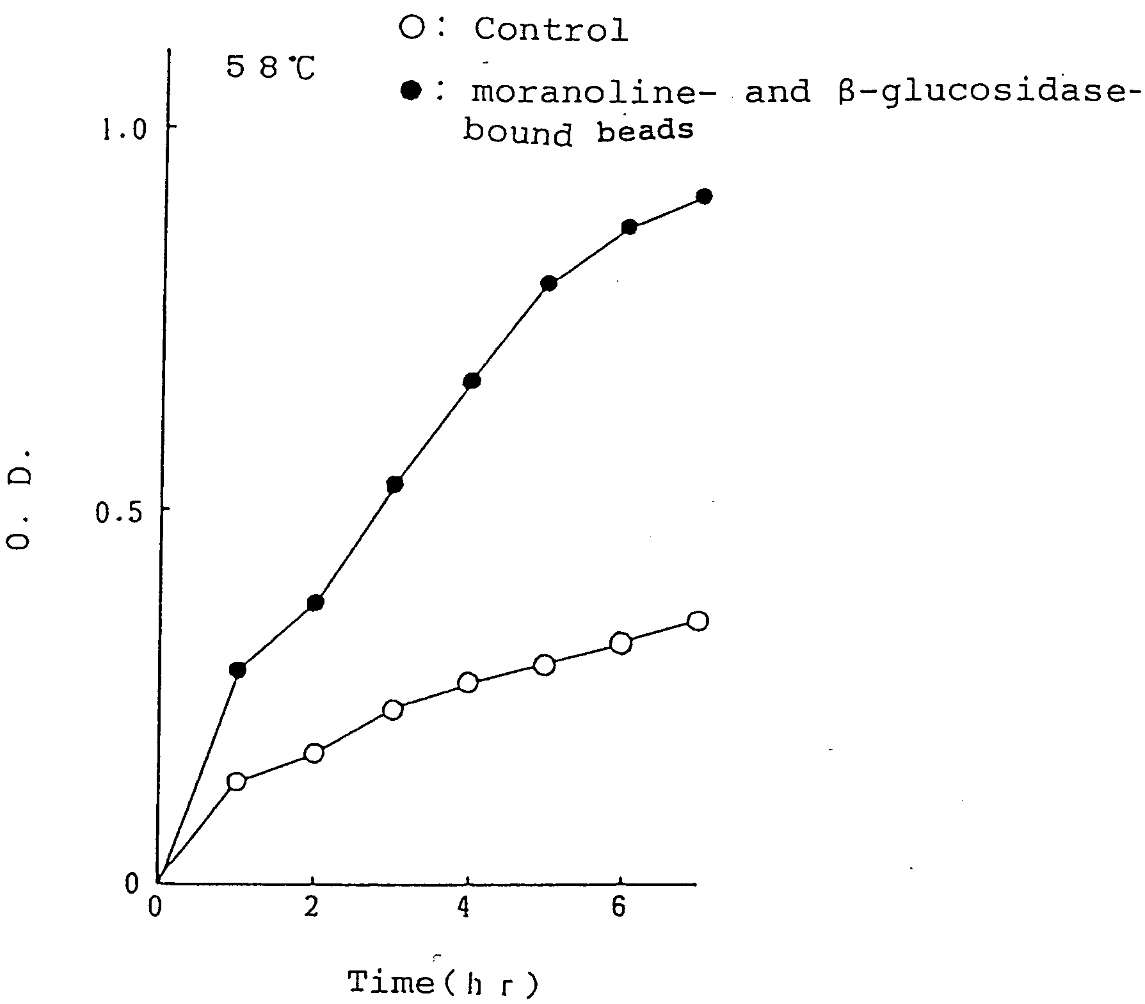

Fig. 2
Residual activity curve for moranoline- and β-glucosidase-bound Sepharose 6B

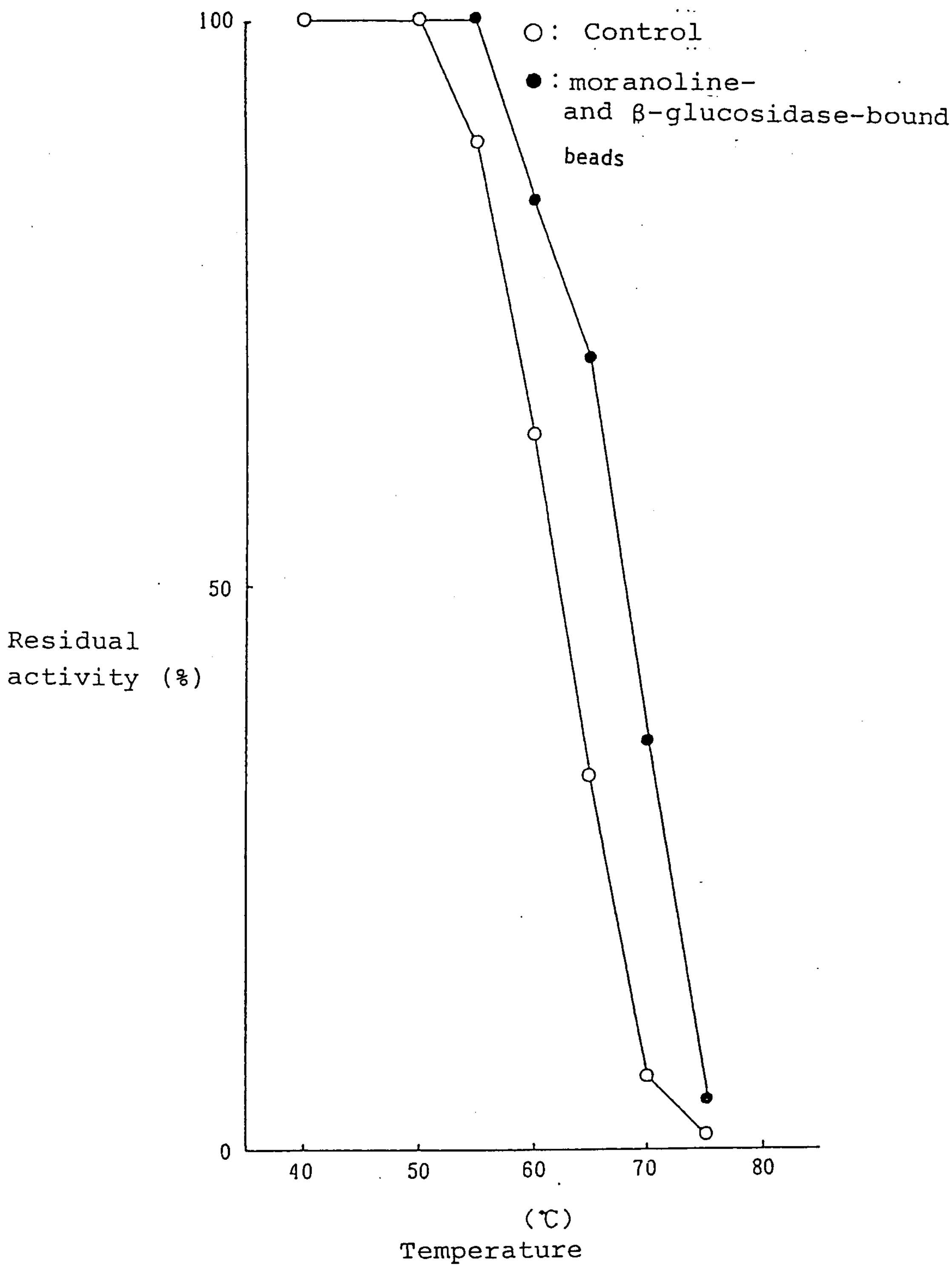

Fig. 3.

Heat stability of N-methylmoranoline- and β-glucosidase-bound Sepharose 6B

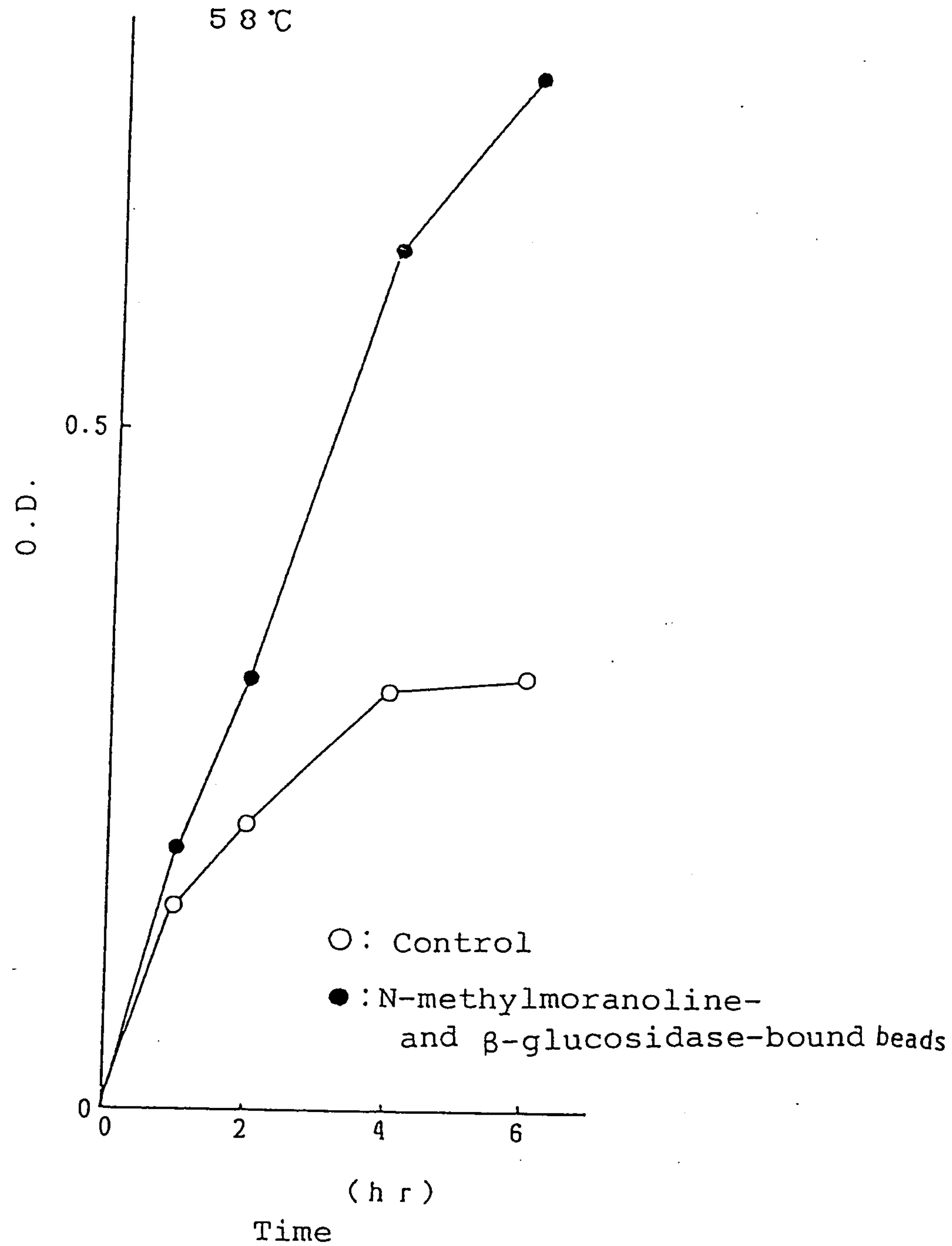

Fig. 4.

Residual activity curve for N-methylmoranoline- and β-glucosidase-bound Sepharose 6B

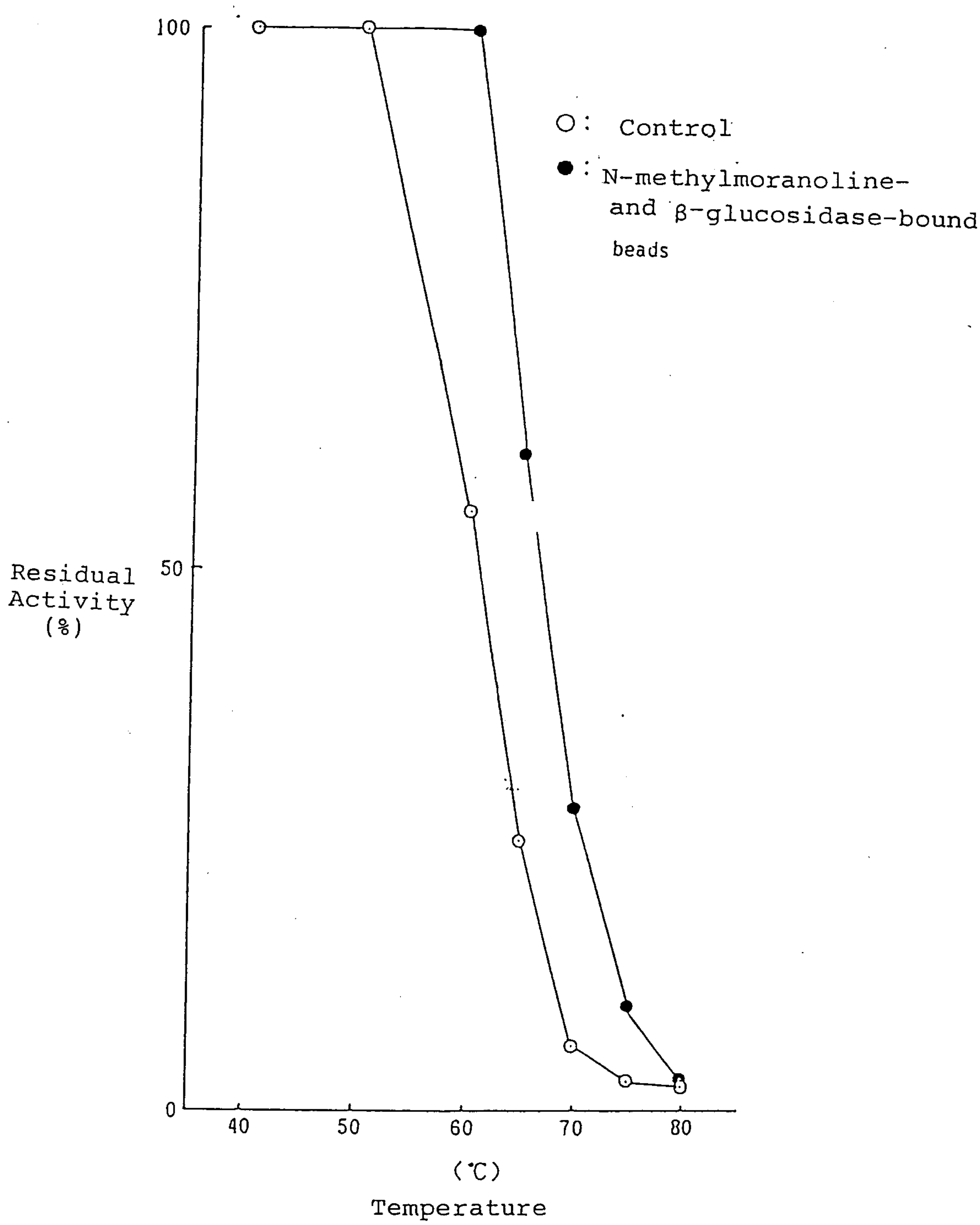

Fig. 5

Heat stability of moranoline- and glucoamylase-bound Sepharose 6B

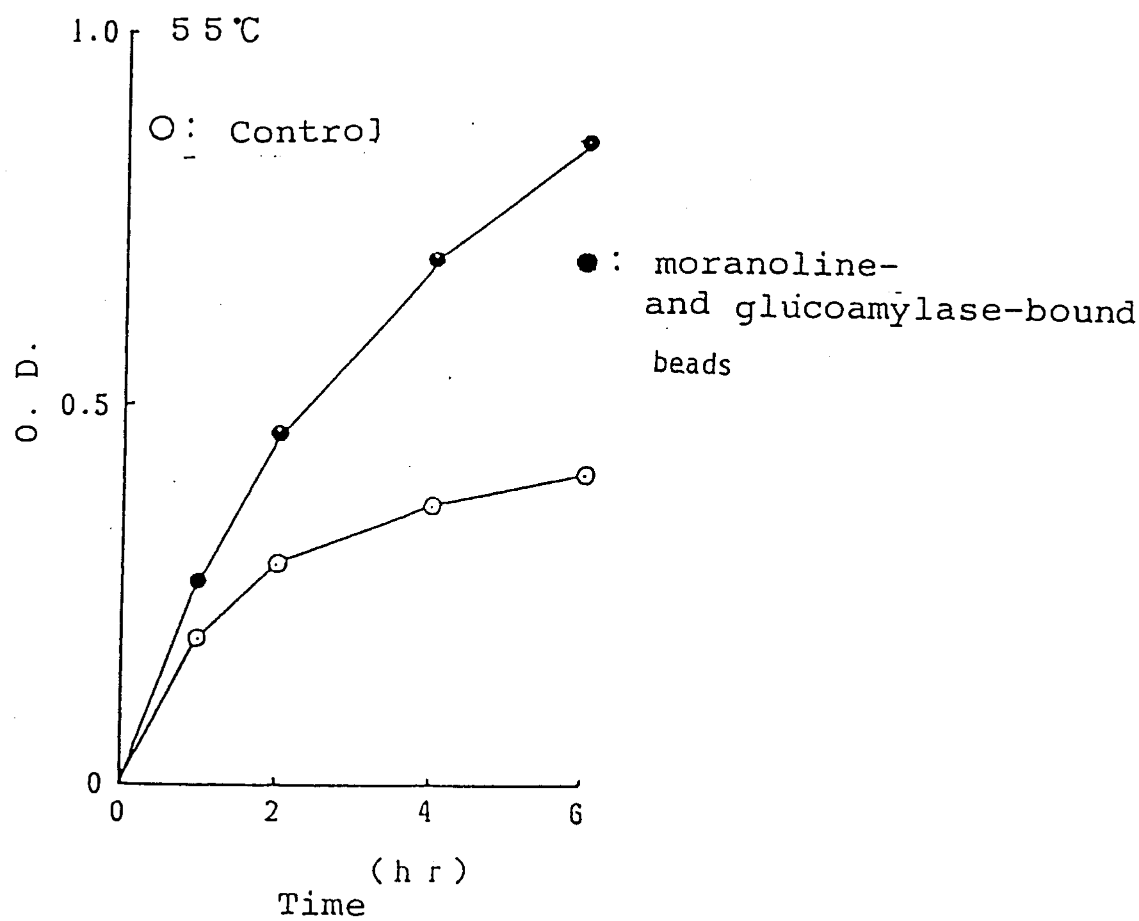

Fig. 6

Heat stability of moranoline- and glucoamylase-bound chitosan beads

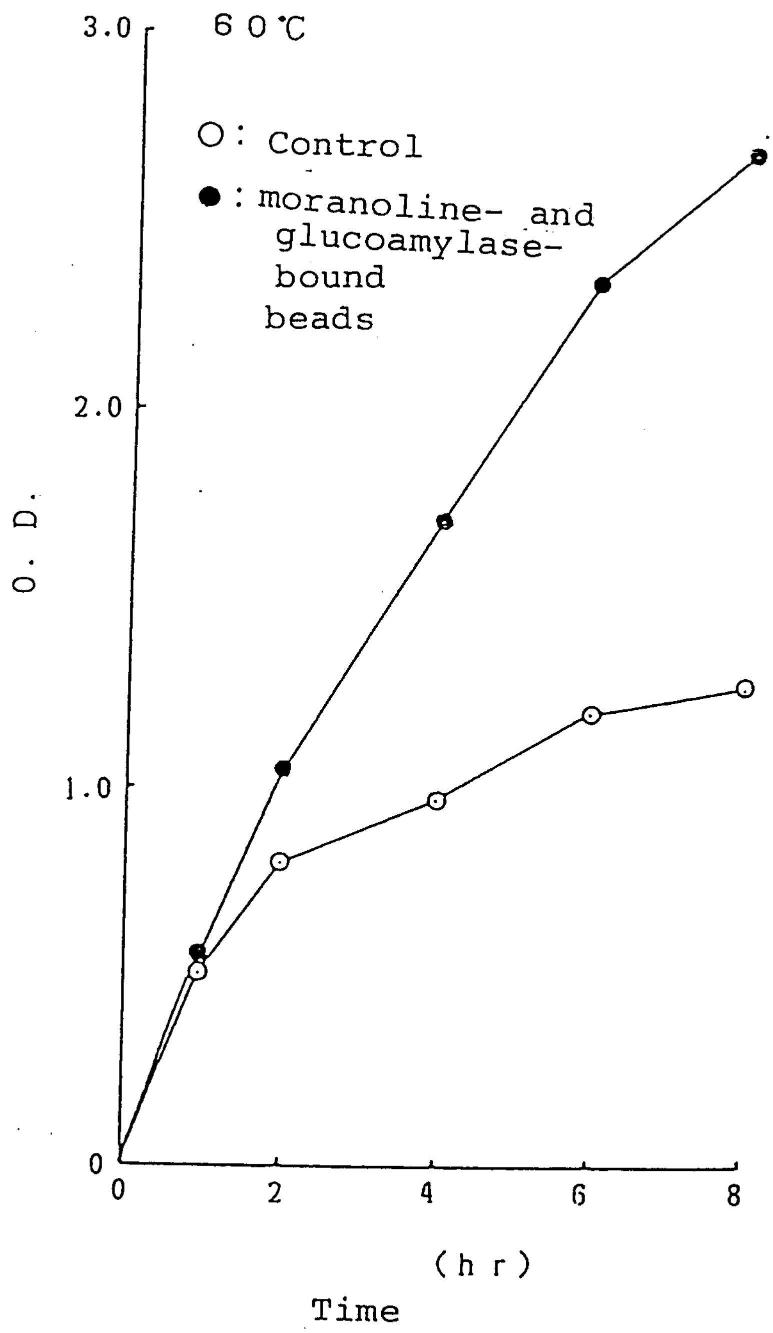

Fig. 7

Heat stability of N-(2-hydroxyethyl)moranoline-bound chitosan beads

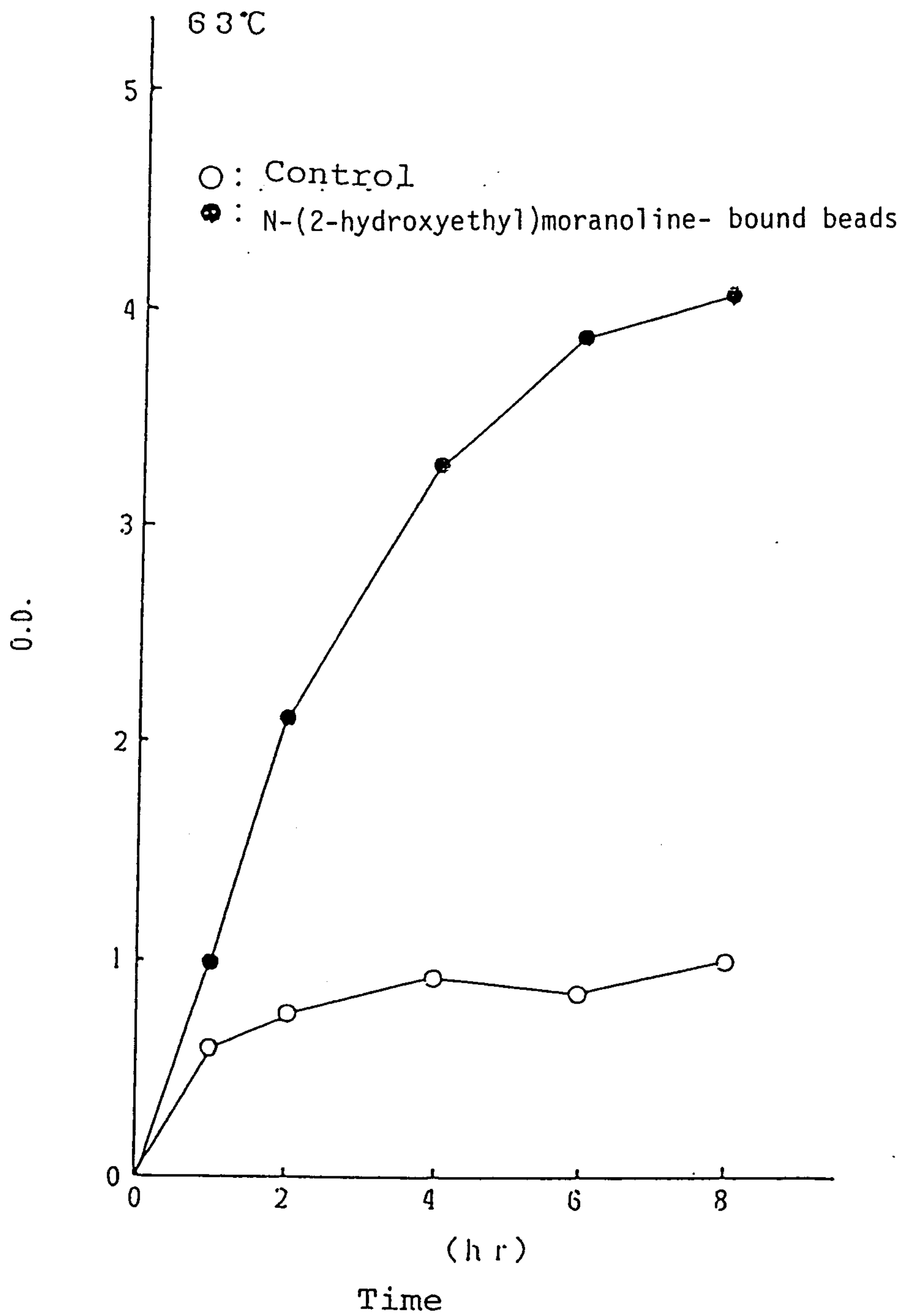

Fig. 8

Heat stability of α-cyclodextrin- and glucoamylase-bound Sepharose 6B

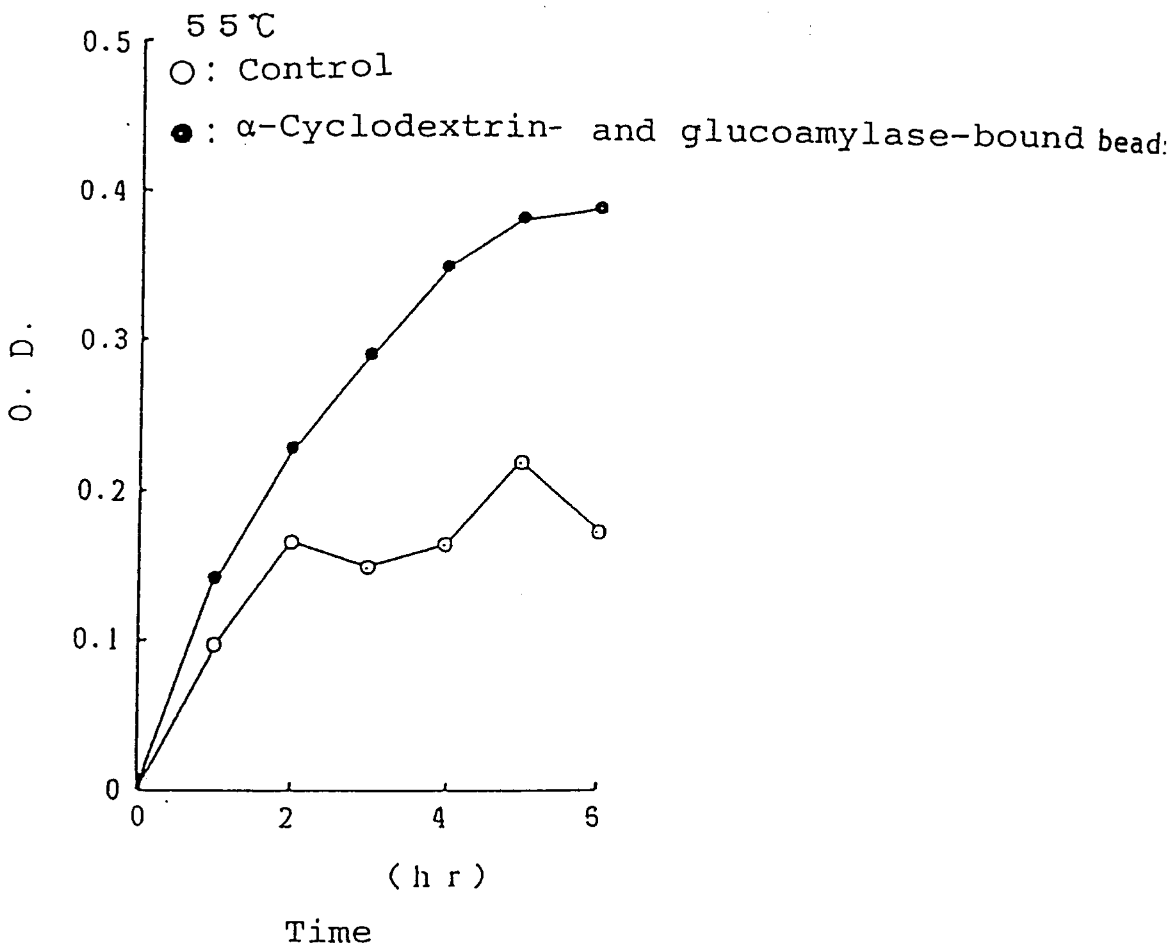

Fig. 9

Residual activity curve for α-cyclodextrin- and glucoamylase-bound Sepharose 6B

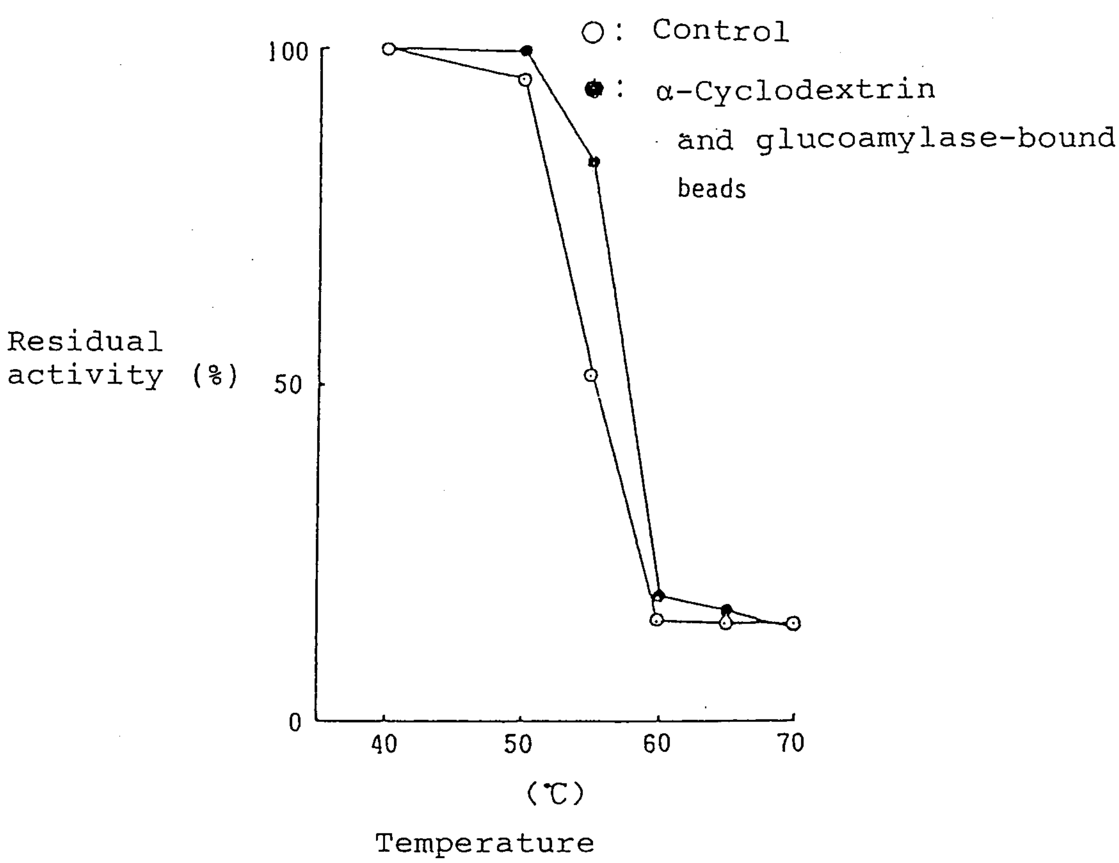

Fig. 10

Residual activity curve for glucosylmoranoline- and CGTase-bound chitosan beads

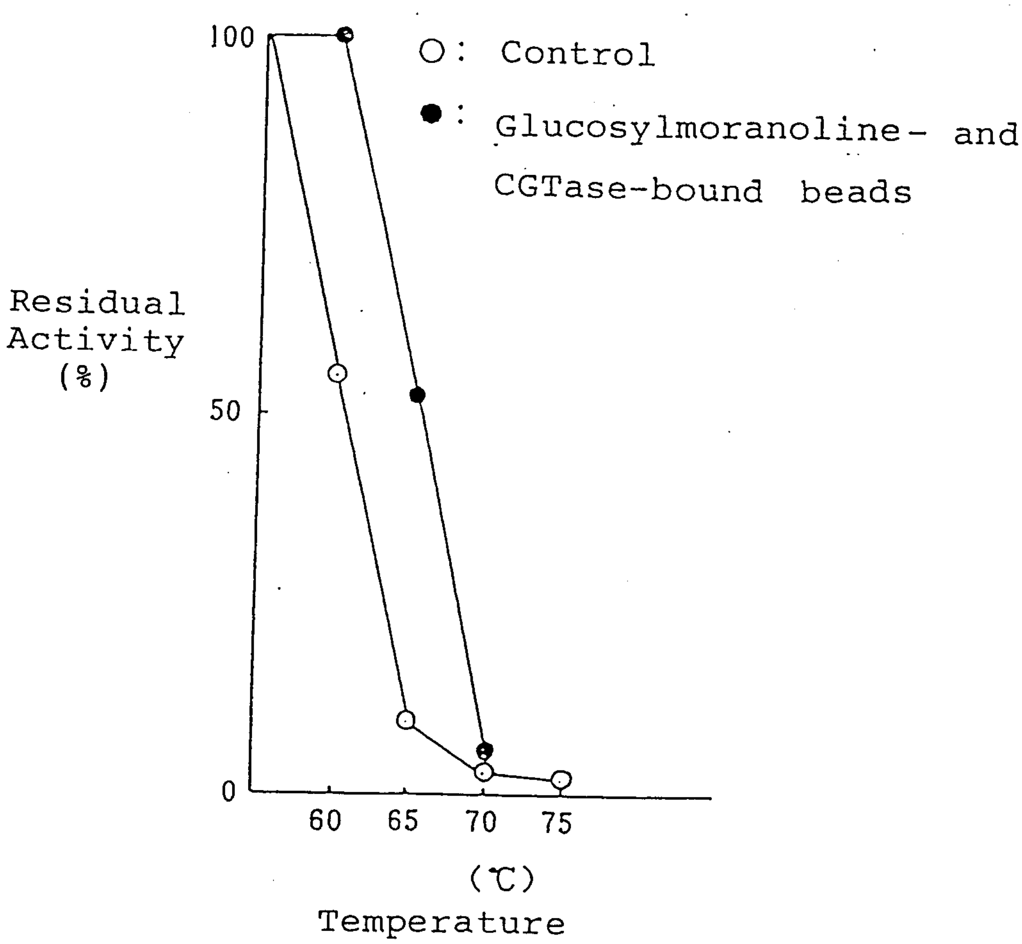

Fig. 11

Residual activity curve for glucosyl-N-methylmoranoline- and CGTase-bound chitosan beads

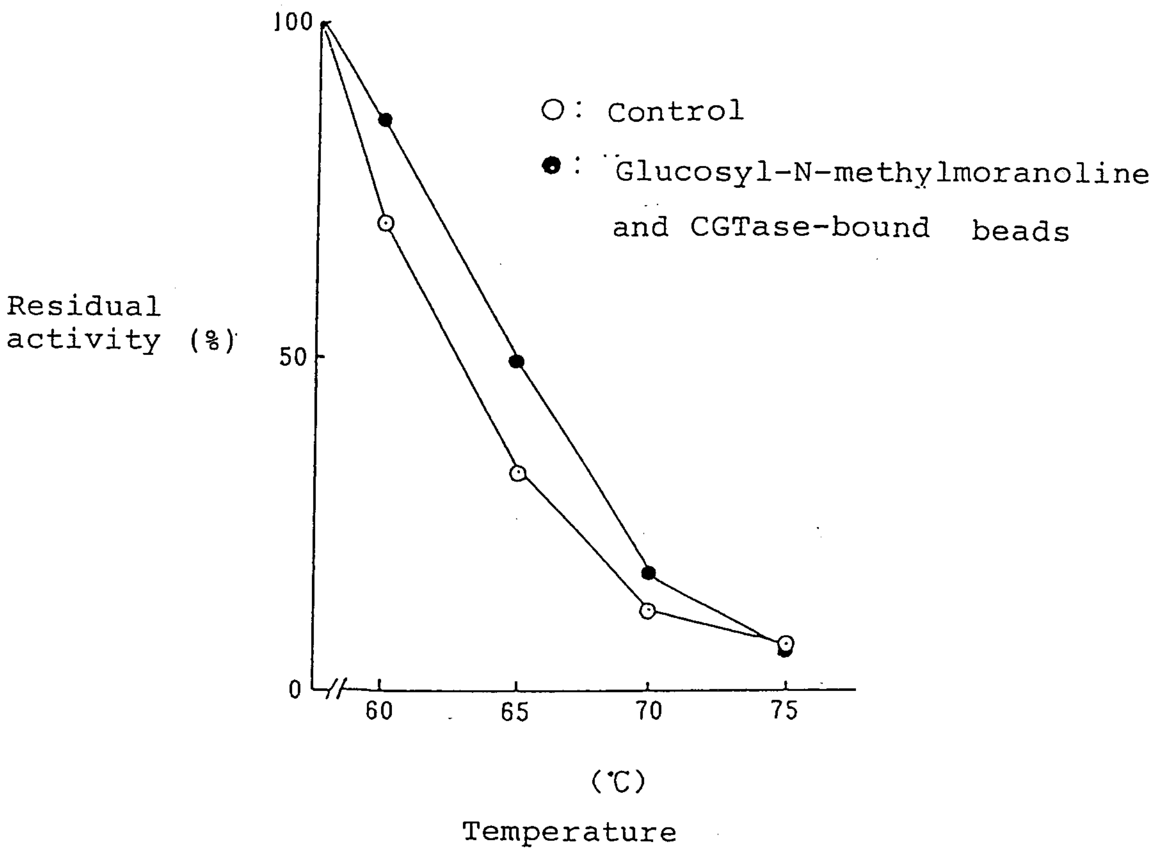

Fig. 11.

Heat stability of 1-deoxygalactostatin- and β-galactosidase-bound chitosan beads

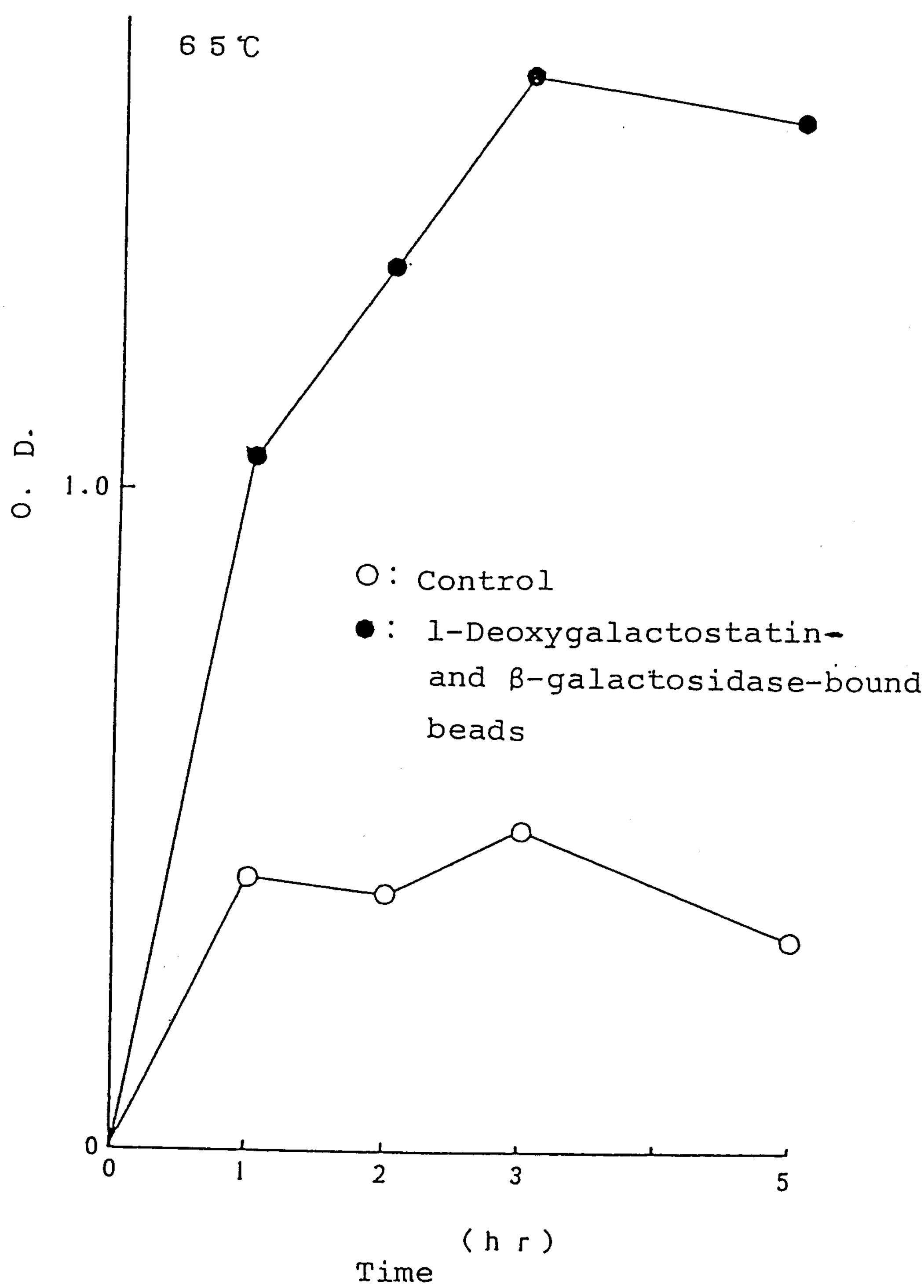

Fig. 13

Residual activity curve for 1-deoxygalactostatin- and β-galactosidase-bound chitosan beads

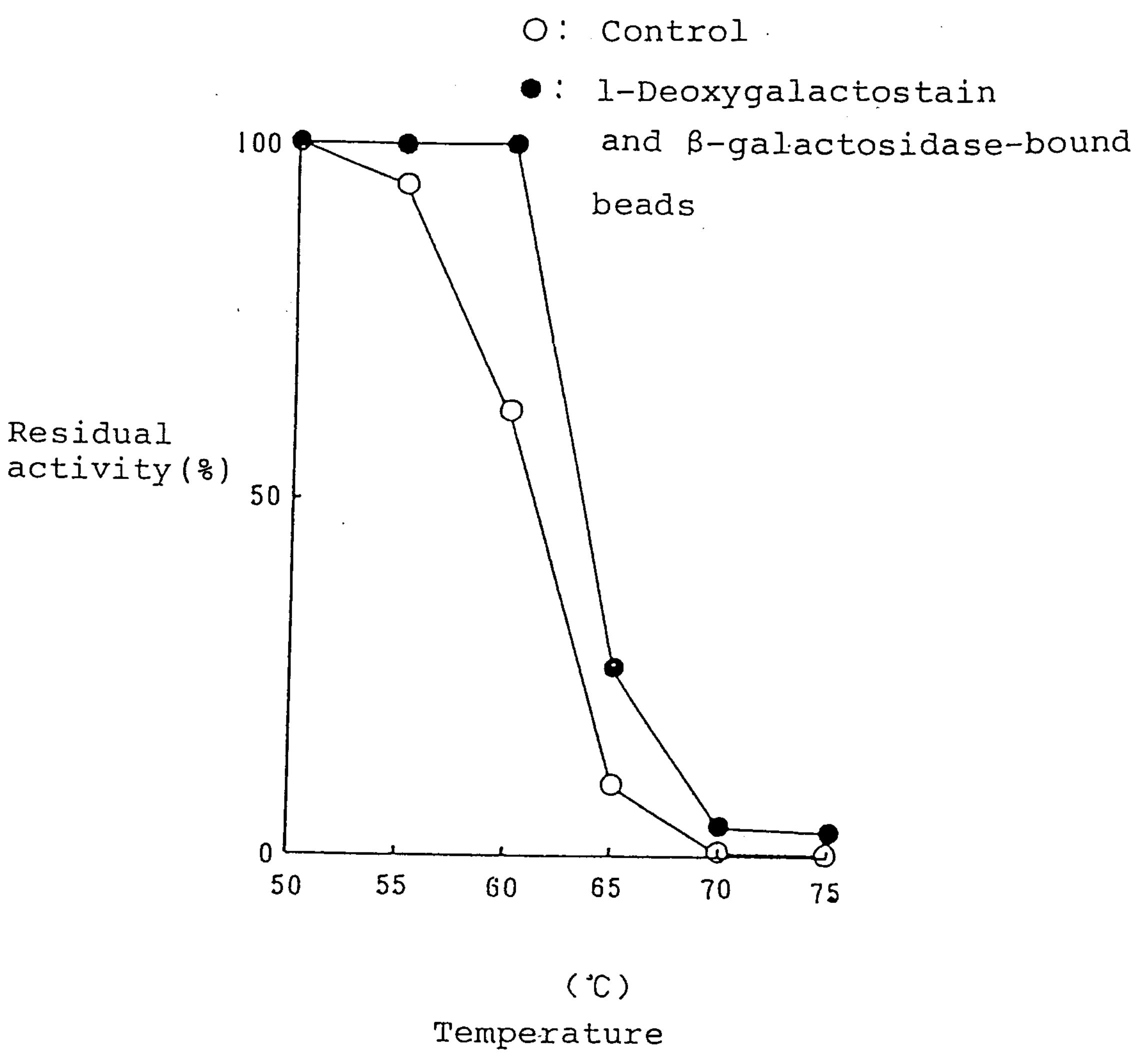

Fig. 14

Heat stability of 1,4-dideoxygalactostatin- and β-galactosidase-bound chitosan beads

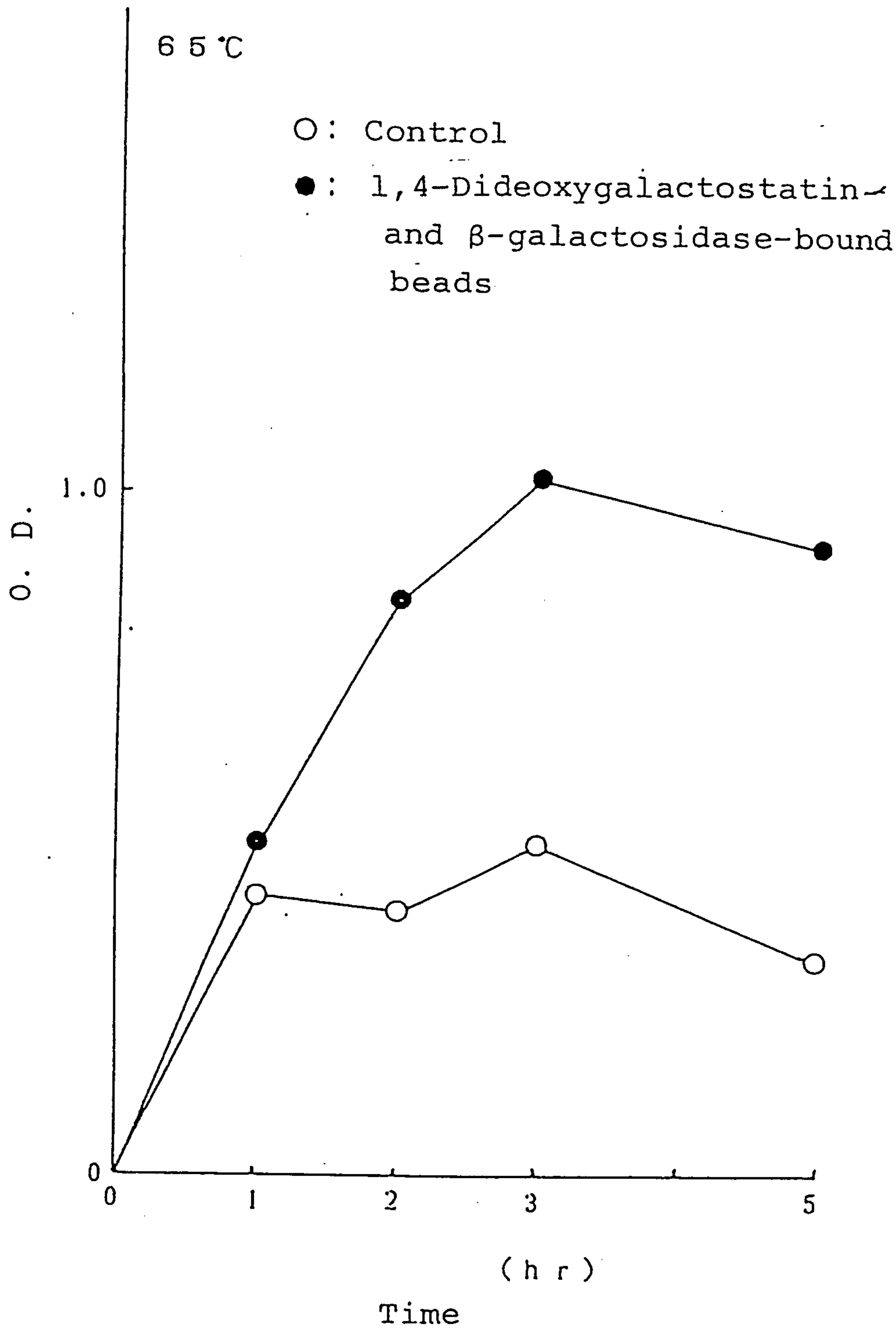

Fig. 15

Residual activity curve for 1,4-dideoxy-galactostatin- and β-galactosidase-bound chitosan beads

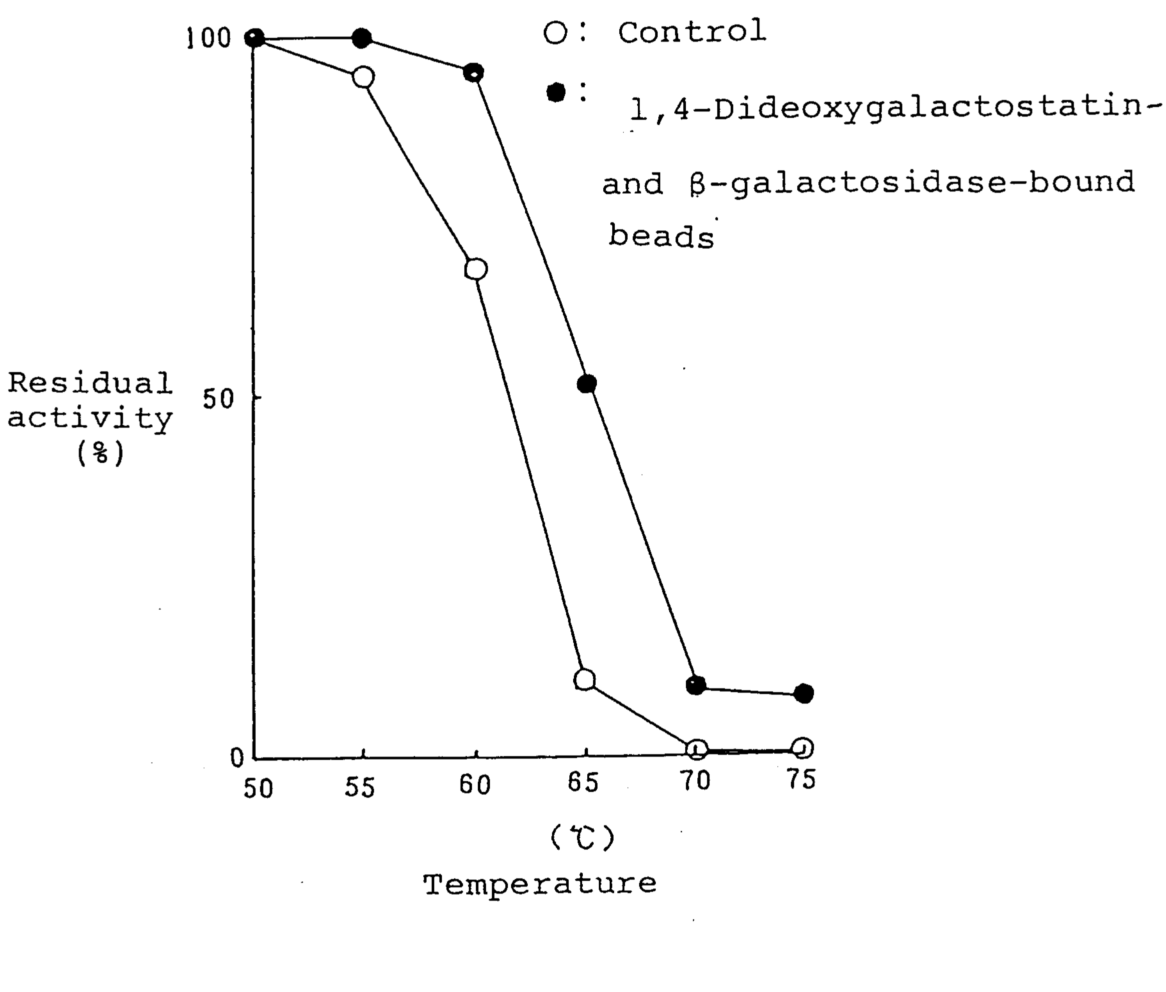

Fig. 16

Heat stability of glucosylmoranoline- and β-amylase-bound chitosan beads

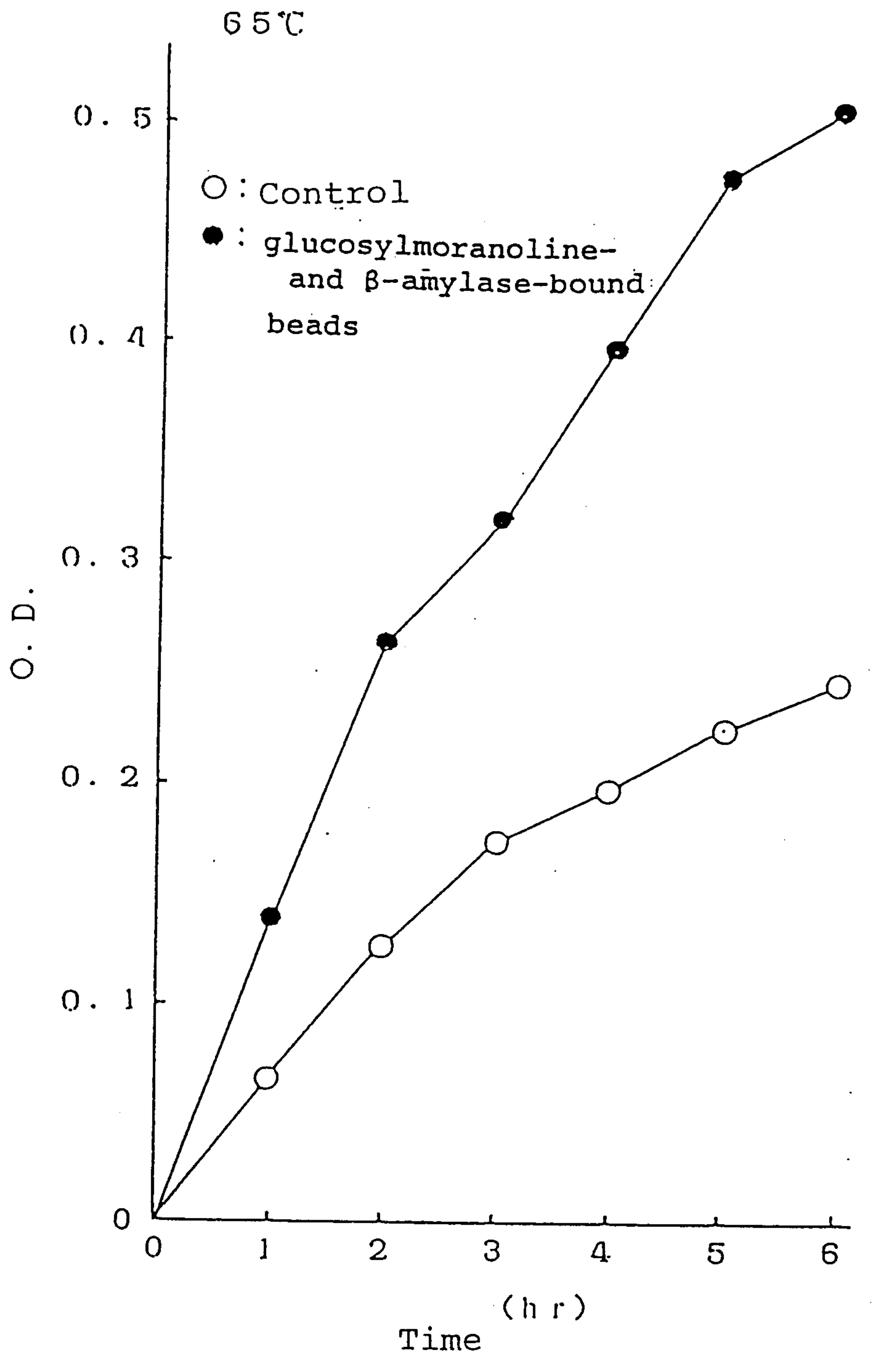

Fig. 17

Residual activity curve for glucosylmoranoline- and β-amylase-bound chitosan beads

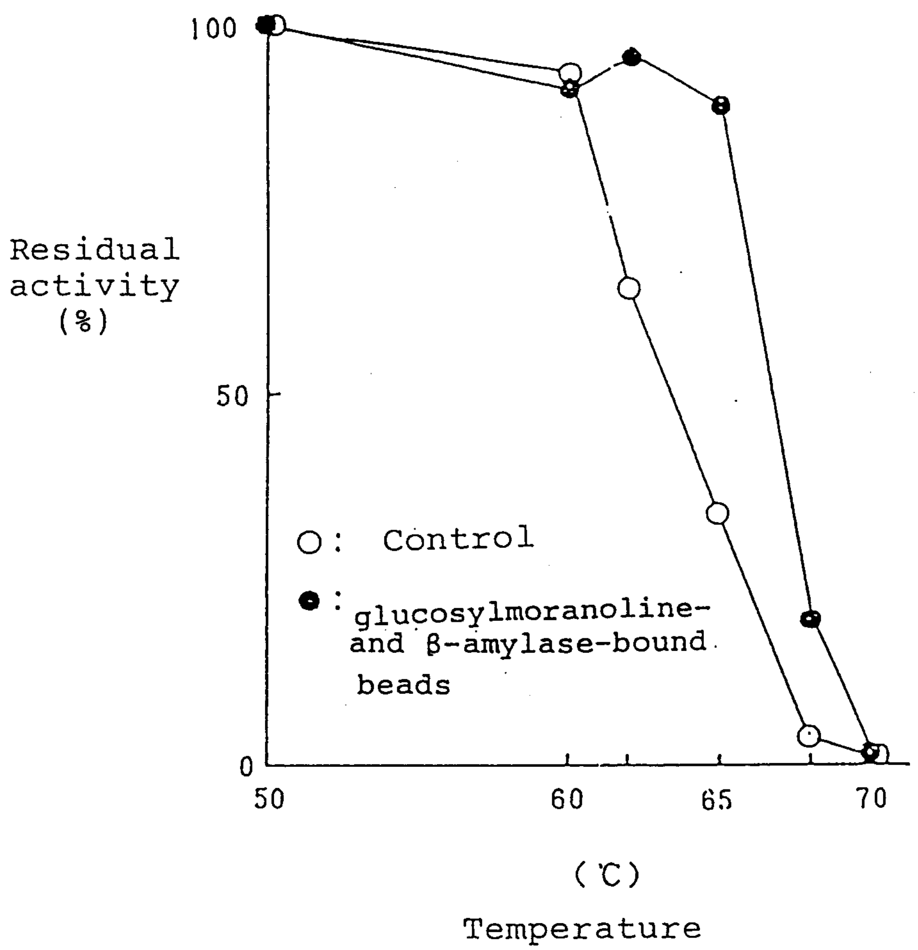

# INTERNATIONAL SEARCH REPORT

International Application No PCT/JP90/01395

**I. CLASSIFICATION OF SUBJECT MATTER** (if several classification symbols apply, indicate all) [6]

According to International Patent Classification (IPC) or to both National Classification and IPC

Int. $Cl^5$ C12N11/00

**II. FIELDS SEARCHED**

Minimum Documentation Searched [7]

| Classification System | Classification Symbols |
|---|---|
| IPC | C12N11/00-11/18 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| X | JP, A, 58-28285 (Teito and Rail Public Co., Ltd.), February 19, 1983 (19. 02. 83), Claim, lines 6 to 19, upper left column, page 3, lines 2 to 4, upper left column, page 5 (Family: none) | 1 |
| A | JP, A, 52-15890 (Unitika Ltd.), February 5, 1977 (05. 02. 77), Claim, line 12, lower right column, page 1 to line 18, upper left column, page 2 (Family: none) | 1, 2, 3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 11, 1991 (11. 01. 91) | February 4, 1991 (04. 02. 91) |
| **International Searching Authority** | **Signature of Authorized Officer** |
| Japanese Patent Office | |